(19) **Europäisches Patentamt / European Patent Office / Office européen des brevets**

(11) **EP 3 528 057 A1**

(12) # EUROPEAN PATENT APPLICATION
published in accordance with Art. 153(4) EPC

(43) Date of publication:
**21.08.2019 Bulletin 2019/34**

(21) Application number: **17860621.6**

(22) Date of filing: **11.10.2017**

(51) Int Cl.:
*G03H 1/00* (2006.01)    *G03H 1/04* (2006.01)
*G01N 15/02* (2006.01)

(86) International application number:
**PCT/BR2017/000124**

(87) International publication number:
**WO 2018/068112 (19.04.2018 Gazette 2018/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **11.10.2016 BR 102016023757**

(71) Applicants:
• **Guedes Braguine, Patrícia**
  **CEP: 37950-000 São Sebastião do Paraíso - MG (BR)**

• **Guern Tecnologia Da Informação Ltda - Me**
  **CEP 13360-000 - Capivari - SP (BR)**
• **Fundação Universidade Federal De São Carlos**
  **13565-905 São Carlos - SP (BR)**

(72) Inventors:
• **GUEDES BRAGUINE, Patrícia**
  **13565-905 São Carlos - SP (BR)**
• **GHIGLIENO, Filippo**
  **13565-905 São Carlos - SP (BR)**

(74) Representative: **Giraldi, Elisa et al**
  **Notarbartolo & Gervasi S.p.A.**
  **Corso di Porta Vittoria, 9**
  **20122 Milano (IT)**

(54) **PORTABLE DIAGNOSIS TEST AND USE THEREOF**

(57)    A diagnostic test system, consisting of hardware and software. Tested samples include, but are not limited to, blood, body fluids, and other components that may be present in the human body. The test comprises a portable camera, software, an artificial intelligence system, a database, a hardware device featuring at least one light source, and a controlled area to keep the sample. Images are captured by the portable hardware system, working together with the artificial intelligence system, for the qualitative and quantitative analysis of the information obtained on the fluorescence and morphology of the sample.

The invention pertains to the technical field of coherent light imaging devices and methods such as Digital Holography (HD) and Digital Holographic Microscopy (MHD), used to test blood and other fluids.

FIGURE 1C

**Description**

**FIELD OF INVENTION**

This invention pertains to the technical field of Coherent Light Imaging Devices and methods, such as Digital Holography (HD) and Digital Holographic Microscopy (MHD), used to test blood and other fluids.

**FOUNDATIONS OF INVENTION**

The holographic technique allows us to record the intensity and phase of a wave front on a physical support and subsequently reconstruct the information by illuminating the recording with a source equal to that of origin. In digital holography, the physical support is a CCD sensor of a camera and the reconstruction is made by means of software, 'virtually' illuminating the recording. Some of the benefits of this technique are: the possibility to provide images, free from quantitative markers and non-destructive application, the characterization of topography and morphological analysis with a minimally invasive procedure, and the non-need to chemically process photographic paper. Furthermore, a holographic image compacts 3D information of the target into a 2D support, allowing its volumetric reconstruction, while in digital holography the software reconstructs the three-dimensional morphology of the sample. It is worth noting that in the case of digital holographic microscopy (DHM), the technique allows us to increase the depth of focus, limited in traditional microscopy by the presence of the lenses.

The holographic microscope (MH) has been used to make different types of inspections, but only recently begun to be used for hematology applications, and to this day remains as diffuse technology, only in research laboratories. In fact, the technique is very effective in terms of image quality, but is quite limited when considering the costs involved and the necessary infrastructure. For example, analysis procedures require highly trained people, available space for the equipment, and even a high initial investment for the purchase of microscopes. In poor and remote areas, this technique is rarely used, and all the benefits that images produced with coherent light could offer in terms of time and assertiveness are unfortunately wasted. Applications related to hematology analyses can significantly improve the quality of the healthcare system. We believe this is an opportunity to be involved in the process of deducing the costs related to the diagnostic equipment, while increasing and expanding its use, in addition to the prevention, detection and monitoring of diseases.

MHD is used to monitor cell cycles and also in microstructure tests, in which three dimensional distributions of particles are measured, including, but not limited to, red blood cells (RBC), and white blood cells (WBC).

Considering also the great technical advances that have taken place in the field of lasers, in camera systems and the gradual increase in the processing speed of computers, new possibilities for the use of this equipment are arising. Currently, professional digital cameras, or even those that can be found on mobile devices (tablets and smartphones) are no longer potential tools, but established components in some experiment settings in scientific laboratories. What we did was to define an application combining microscopy techniques with an artificial intelligence system, capable of performing analytical predictions.

The prototype explores some properties of holographic microscopy, fluorescence microscopy, and an artificial intelligence system, constituting a portable and compact imaging test. From an operational point of view, some elements were strategically inserted in the setup for the performance of qualitative and quantitative inspections. The automatic counting and categorization of cellular elements, as well as the possibility of using videos to collect data extracted from the blood flow were also advances integrated into the development of the prototype. The disease prediction system is based not only on data collected in a single test, but also on the history of tests carried out using the device, which positively contributes to an early diagnosis. The process of monitoring the evolution of circulating tumor cells (CTCs) can also benefit from this approach, which is safe, minimally invasive, and painless.

**STATE OF THE ART**

Among the advances made in the integration of mobile technology to laboratory applications, some progress was made in the attempt to integrate mobile phone cameras with diagnostic tests. For instance, Aruan et al. showed that telephones (iPhones and Androids) with over 05 megapixels (MP) are almost able to reach diffraction-limited resolution over a wide range of applications, including those that are relevant for cellular imaging, in Quantitative Imaging with a Mobile Phone Microscope, PLOS One, www.plosone.org, Vol. 9, Issue 5, (2014). In another example, Pham et al. has developed a highly sensitive instrument that operates in real time without human intervention. Pham et al. Tests in Real Time Blood Testing Using Quantitative Phase Imaging, PLOS One, www.plusone.org, Vol.8, 2nd Edition, (2013). Breslauer et.al reported the development of a high-resolution microscope peripheral for mobile telephones with cameras in *obile* Phone Based Clinical Microscopy for Global Health Applications, PLOS One, Vol. 4, Number 7, (2009).

In one mode of the invention, the system is designed to run portable tests on blood, cellular components, and body fluids, and comprises: artificial intelligence software for counting, classification and analytical predictions; cloud-based software for image reconstruction; a database; a camera; a device containing at least one light source in its interior, and a proper space to insert the sample; a filter to decrease the intensity of the laser; a second filter for capturing the fluorescence.

In another mode of the invention, there is a method to monitor the health of an individual that produces a history of all information obtained through the use of the IG11 test. This process comprises: sending the blood test image(s) to an artificial intelligence system; cross-checking the information of the databases with the images sent; the study conducted by the Artificial intelligence system for the detection of anomalies, similarities, the elaboration of a report, and the sending of the qualitative and quantitative characterization to the user.

In another mode of invention, there is a method to test and monitor circulating tumor cells (CTCs) and the evolution of patients who have a positive diagnosis for the presence of these cells in the blood. This method includes periodic testing using IG11, where any image extracted using the device is sent to the artificial intelligence system using the internet; image categorization; recognition and listing of information on anomalies, similarities, crossings of tumor lines; and the issue of a quantitative report.

## SUMMARY DESCRIPTION OF THE DRAWINGS

Figures 1A, 1B, and 1C provide a view of the device, based on its form of realization. Removable parts are also indicated.

Figure 2 shows an alternative device setting, according to another form of implementation. Removable parts are also indicated.

Figure 3 shows a schematic picture of the operation of the system.

Figure 4 illustrates the logic of the analytical prediction system and the anomaly mapping.

Figure 5A provides the screens that will be used as part of the user interface before entering the sample into the device.

Figures 5B and 5C show how test results will be presented to the user through the application interface or by direct access to the user area on the site.

Figures 6A and 6B show consecutively reconstructed images from a digital hologram.

Figure 7A provides an image of the components of a human blood sample.

Figure 7B illustrates an enlargement of one of the blood components seen using a filter and a LED light.

Figure 7C illustrates an isolated component of the blood.

Figure 7D illustrates image 7C after receiving a non-holographic 3D treatment and reconstruction.

Figure 8 schematically illustrates new possibilities for health monitoring.

## DETAILED DESCRIPTION OF THE DRAWINGS

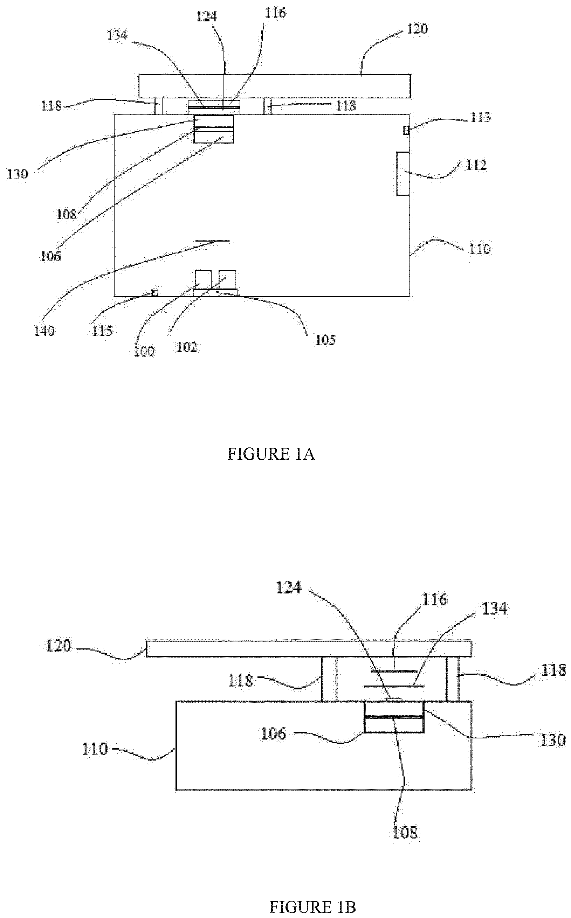

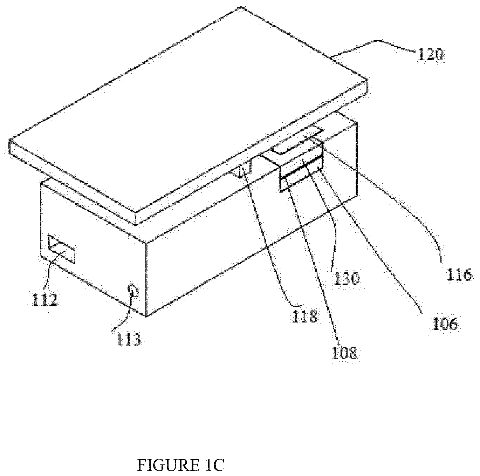

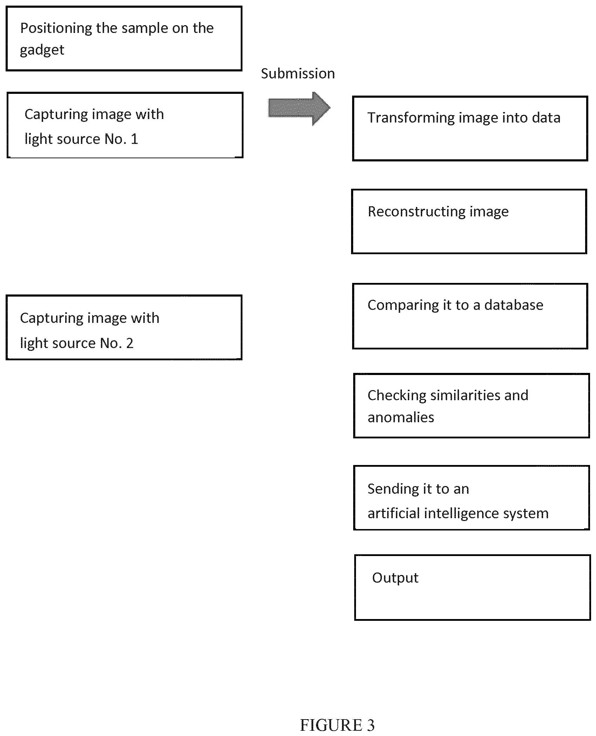

Figures 1A, 1B, 1C illustrate a view of the hardware device. The device consists of a rigid structure (110), which must be supported on a flat, stable and non-vibration surface. The sample (108) must be deposited in a controlled place on a prepared slide (106), which may be made of glass or polymeric material. This blade may contain in its constitution or on its surface, substances known as chemical markers. Immediately over sample 108, there goes a second blade (130), which presses it. The rigid structure (110) may contain one or more inputs (112) and (113), which serve as connection ports to external devices and/or for charging energy. Entrance (112) may be a USB port and entrance (113), an entrance to another type of cable. In the case of the USB port, the device can be connected to a mobile phone, tablet, notebook, or any other device for the transmission of data to the system architecture detailed in Figure 3. In Figure 1A, light source (100) may be a white light source or even a coherent light source. When a Coherent Light is used, it may vary from visible to infrared. It is also possible to have a combination where there is a second light source (102), which can also be white or coherent. Light sources may be on a platform (105) where it is possible to select the type of light to be used during the test.

The sample as indicated in Figures 1A, 1B and 1C, shall be inserted in a controlled place and necessarily undergo a slight compression, which may be caused by the deposit of a second blade on its surface or by a drag caused by the fitting of the sample into the holder. The sample may be blood, urine, bodily fluids or some other component that is not originally part of the human body's Constitution but is present in it. For example: viruses, some bacteria, and nanoparticles.

The casing of the device illustrated in Figures 1A, 1B, and 1C may have different geometries and may contain a location for support insertion (118), for mechanical stabilization of the camera or mobile device. Because there is a great need for System Stability Control, additional supports can be added. Points (118) can also be used to fit the device in IG11. After the capture of the images, it is necessary to send them over the internet via Wi-Fi, cable, Bluetooth, or even by downloading these images on a computer. In this case, these images shall be uploaded to the online platform, with all related information, instead of the application (which has the same features).

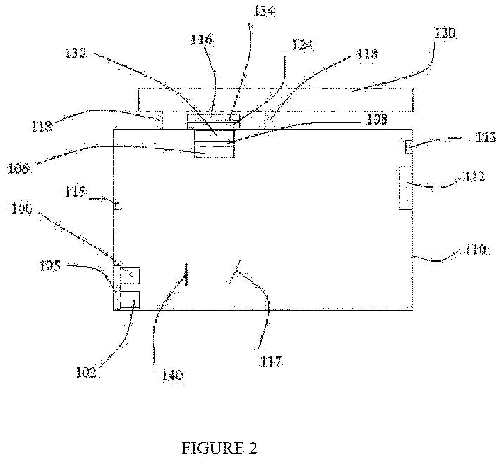

Figure 2 shows a second configuration of the device where the light source is schematically directed to a mirror located between the source and the sample. This mirror aims to divide the beam from one of the light sources. A portion of the beam travels a straight trajectory, suffering a low diffraction as the reflected beam travels a second trajectory through the sample. Both secondary beams Intercept in the sample. The lens shall be positioned after the sample, opposite to the laser.

The settings laid out in Figures 1A, 1B, 1C and 2, show necessarily the participation of at least one lens (116) external to the components of the system, and that may be part of the physical body of a smartphone (120), notebook or tablet, but necessarily allows a higher resolution than 5 megapixel.

The system may also contain the lens (124) between the sample (108) and the device (120).

Still on figures 1A, 1B, 1C and Figure 2, the rigid part (110) and some of the components of IG11 can be reused numerous times without risk of contamination since the slide (108) containing the sample can be completely removed. It is important to note that after the use of each blade, it should be completely discarded and cannot be reused. The device may be accompanied by only one or several spare blades.

In Figures 1A, 1B, 1C, and Figure 2, the sample (108) shall be arranged in a controlled location, marked for the correct placement of lighting and, if applicable, of chemical markers. After inserting the sample, the capture of the images must be started.

As described in more detail below, it is not necessary to have any different software besides the one that comes with the device, with the function of optimizing image capture. The camera to be used, which may or may not come with flash and automatic focus options, should preferably have the Auto-Focus functionality disabled, as well as the flash or any other that can change the lighting conditions of the sample.

The IG11 device can count cells or batteries stored in (115) or alternatively can be charged by doors (112) and (113), or by connecting in-line with another device, provided voltage specifications are respected. The device described here can be used in conjunction with different devices such as smartphones, tablets and digital cameras, since the existence of an external prompt is not required for its use. What is required is the use of a device with a camera with a capacity for at least five megapixels.

Also on figures 1A, 1B, 1C and figure 2, the devices described therein may contain a filter (140) with the function of attenuating the light source before it reaches the sample. A spatial filter is essentially a beam convergence device coupled with a filter. The filter, or pinhole in our configuration, is used to remove interference patterns in a laser beam caused by diffraction of dust, hair, lens imperfections, etc. which are part of any optical system. Diffraction interference degrades the laser beam by producing phase and amplitude variations.

The schemes represented by figures 1A, 1B, 1C and Figure 2 may contain one second filter (134) with a different function from that of filter (126). In that case, the filter is between the sample and lens (124), and can be deposited directly on slide (114), pressing the sample. It may also be attached to structure (118) belonging to the support place between the sample and the lens of the device. This filter can be removable and replaceable, depending on the type of sample to be tested.

The devices depicted in Figures 1A, 1B, 1C and Figure 2 show the configurations of the hardware used for capturing the images. For the detection of white and red blood cells, the sample must have a volume of less than 1 milliliter. For CTCs detection, it is necessary to have a volume below 5 milliliters.

Figure 2 shows a configuration where a mirror (117) was inserted into the system to make with let the beam travel two different paths and be gathered back into the sample. This configuration causes the hologram to be rebuilt with a high quality gain.

Lens (116), used during the tests, was produced in order to be ultrafine so that there was an increase in the level of compaction of the system and a reduction in the volume of material to be discarded. The system can display one or more ultrafine lenses in its composition, in different locations, which can even be inserted directly over the lens surface of devices such as smartphones, tablets, and computers. In such cases, the lens shall be positioned centrally, enabling its full use.

As shown in Figure 3, the images taken by the IG11, here represented by Figures 1A, 1B, 1C and Figure 2, are sent and only then processed by the cloud-based system or an external physical server. On mobile devices, only basic processing is performed, applied to any image. The functionality explored in these devices is that pertaining to imaging. In this way, after sending it to a cloud-based system, the information begins to be extracted from the images collected.

Figure 3 represents the operation of the system as a whole, taking into account from the process of capturing images, enabled by IG11, to sending the result to the user. The software system not only serves as an interface but also for the conversion of images into data, holographic reconstruction and for the qualification and quantification of the sample.

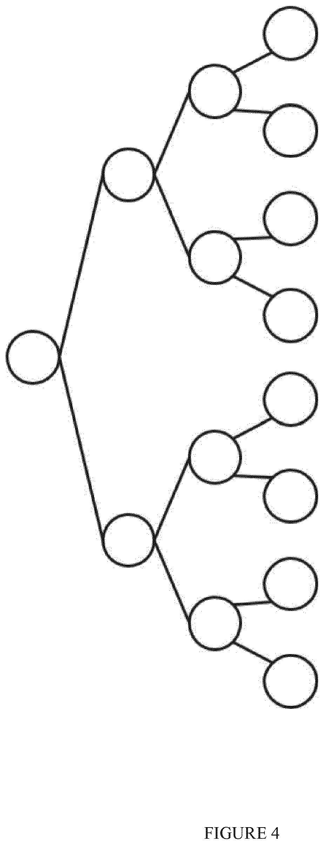

Figure 4 schematically describes an analytical prediction system using decision trees and an anomaly detection system. The algorithm used was developed to operate in conjunction with an automatic counting system, without human intervention, with invariant properties in its hierarchical representation layers. This method obtains a complex and abstract representation of the data in a hierarchical way using multiple layers of nonlinear processing. The algorithm uses Leo Breiman's CART decision tree models in addition to components that make it possible to apply a large number of parameters such as volume, frequency, dimensions, morphology, and topography. The algorithm searches for similarities, as well as anomalies. In this way, any values that are outside the reference standard are identified as anomalies. In this second decision layer, the algorithm verifies what is standard within the anomaly.

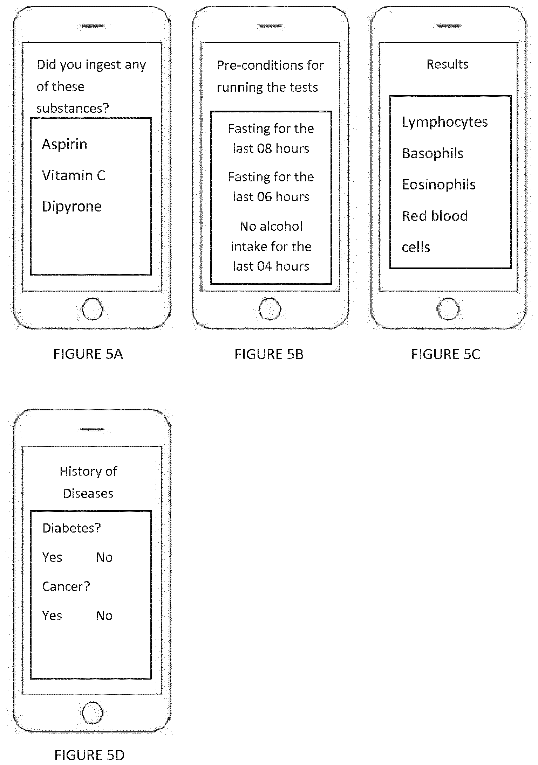

Figures 5A, 5B, 5C show the screens from the menu of a smartphone. As in a clinical analysis laboratory, some information must be provided so the test results can be true to reality. For this, it is necessary to fill in answers to questions about any medicine ingested, and whether or not water and food deprivation was prescribed during the period preceding the test.

The 5D image schematically shows one of the application's interfaces. The purpose of this is to add data that may contribute to a more detailed analysis of the user's health. The form presents simple questions concerning family medical history, besides the existence of any specific condition.

## EXPERIMENTAL RESULTS

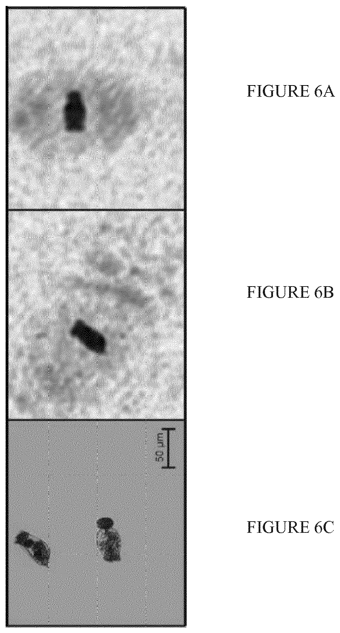

Experimental configuration 1A was used to obtain Figure 6, for which 2 lenses were used in conjunction with a portable device camera with at least 5 megapixels.

In Figure 6C, we compare the ROI magnification with the images produced from one equivalent sample with FlowCAM. The FlowCAM was equipped with a 4x objective lens. A second comparison was made with a phase 3x microscope, in a configuration implemented with Rodenstock lenses (f#5 and 15 mm diameter), and a frequency filter added to the online configuration. This configuration offered equivalent results to those obtained with digital holographic technique using online configuration (IHL) and FlowCAM images, which has a resolution of only 200 micrometers due to the low depth of focus. Zoom and self-Focus Features can make IHL very competitive when compared to other techniques. The greatest advantage is in terms of the Deep of Field (DoF), which can scan and digitally reconstruct the image with high precision, comparable to that of microscopes.

The configuration of Figure 2 can ensure an improvement in image quality in some aspects, such as deletion in ghost image suppression.

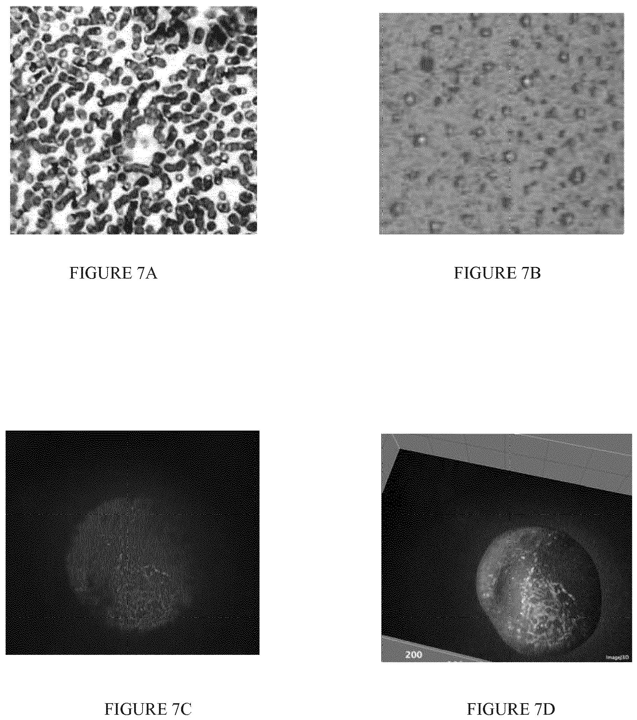

As can be seen in Figures 7A, 7B and 7C, the use of the holographic system brings digital benefits, as the automatic zoom and focus enable a high quality reconstruction, aggregating in terms of morphology and topography of the sample. The online configuration used provided comparable results to DHI and FlowCAM images.

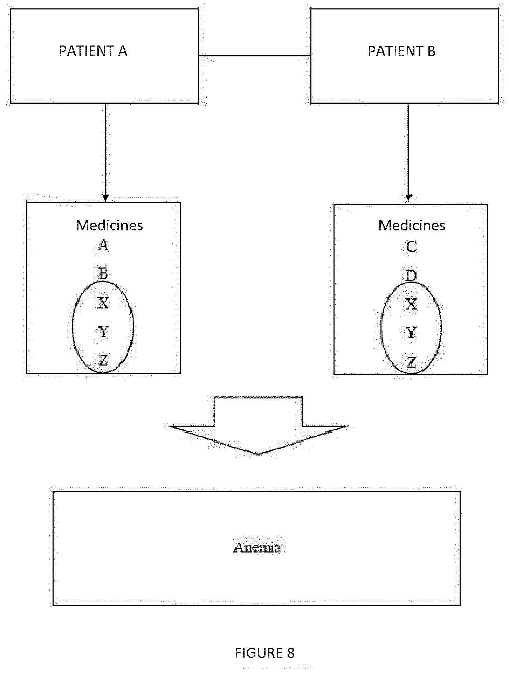

Figures 8A, 8B, 8C, and 8D were obtained by means of an on-line configuration featuring a coherent light source with a wavelength equal to 660 nm, while an output power of up to 30 mW reached the sample on the support. The distance between the laser and the collimating lens was fixed at 4.8 cm. In addition, measurements were made with a laser output power of up to 10mW and a wavelength equal to 532 nm, as well as a laser diode with an output power of up to 5mW, and 405nm wavelength. The camera used was the webcam Logitech HD C270, together with two ultrafine portable lenses and an iPhone 5s mobile device, made by Apple. An interfering filter between the laser and the sample was used to remove unwanted energy spikes, and reduce laser power. In this way, only the central maximum of the diffraction pattern passed through the system. The sample components range from 2 to 20 micrometers.

Still on figures 7A, 7B, 7C, and 7D, the distance between the lens and the sample was between 2mm and 5cm, with the help of a support built to fix the camera on the setup and maintain the focus of the lens of the system. A lens was placed in direct contact with the webcam to increase the optical capacity of the system. The configuration was performed in order to make possible the capture of data for hologram reconstruction as well as the fluorescence of the components to be tested. The method used in fluorescence microscopy has benefits different from those of the microscopy by holography, and when combined in the same system for data collection, in conjunction with the synergy between software and hardware can minimize the chances of errors and instabilities that may be present in the system such as vibrations, images ghosts, or even distortions. The automatic focus makes it possible to compensate for the lack of mechanical stability even under conditions of lower technical control.

Still on how images 7A, 7B, 7C, and 7D were obtained, great care was taken with the lateral resolution, as the distinction of the different cell types also depends on it. This resolution relies on various experimental parameters such as Light Source, sensor definition, distance, object size, location and field of view. The tests were mostly carried out using the wavelength centered on the red region of the electromagnetic spectrum, but note that the Blue diode allows the optical system to be limited to the resolution imposed by the pixel size. Although this aggregate more in quality, it makes the configuration more costly. Selecting the proper wavelength is important because it plays a significant role in the separating power of the equipment.

The image is reconstructed using the Gabor formula for holography. R represents the reference wave front, R represents the wave front that crosses the sample and brings the information to diffraction.

$$I_H\left(x, y\right) = \left(R + R'\right)\left(R + R'\right)^* = |R|^2 + |R'|^2 + R^* R' + R' R^*$$

The dilation factor is determined by the axial distance. For long distances, the dilation increases. For small particles we can write the above equation with the following analytic expression:

$$I\left(r\right) = 1 - \frac{2\pi a^2}{\lambda z} Sin\left(\frac{\pi r^2}{\lambda z}\right)\left[\frac{2J_1\left(\frac{2\pi ar}{\lambda z}\right)}{\frac{2\pi ar}{\lambda z}}\right] + \frac{\pi^2 a^4}{\lambda^2 z^2}\left[\frac{2J_1\left(\frac{2\pi ar}{\lambda z}\right)}{\frac{2\pi ar}{\lambda z}}\right]^2$$

In order to record the minimum diffraction pattern to reconstruct the object, the distance between the sample and the chamber plays a major role, represented in the formula by parameter z. In fact, the dilation factor is determined by the axial recording distance. For the greater distance, the dilation increases.

The synergistic coordination between hardware and software brings benefits in terms of speed and reliability, in regard to the data resulting from sample analysis. In 7A, it is possible to observe white and red blood cells components, identified by the system. The image captured here used low coherence light, LED light. We used a double filter and monochrome light to obtain image 7B, where white blood cells, red blood cells and platelets are again depicted. Image 7C depicts an isolated blood component, and was obtained using coherent wavelength light in the red region of the electromagnetic spectrum, so that holographic reconstruction of the component could be carried out. Image 7C received filter treatment and was rebuilt in 7D.

8A images made use of a system of interpretation and transformation of these into data. This brings enormous benefits in terms of the quantification and qualification of the samples. The sorting of images together with machine learning technique makes room for the categorization of cells. As part of the process, we resort to software to process the data captured by the hardware. The use of machine learning or other similar techniques aims to increase the speed and decrease the error rate arising from visual inspections. Another advantage of using this type of intelligence and making a hardware system that is dependent and connected to software, is to establish relations between data that often do not seem to be connected. The critical factor of our system is the choice of parameters and data to be entered. The analysis of multidimensional data also brings advantages on how to check for the occurrence of pre-disposition for the development of some diseases. For this reason, the need to build a database with information in line with adopted parameters is key.

Among the benefits of employing this technique are the possibility of applying it to different types of samples, the use of a robust system, able to point out connections that may not be obvious to human inspections, the chance to cross a large volume of data, and to adjust the mathematical model that describes the system.

The system used herein obeys a self-learning logic, and was initially provided with a database following certain actions, which is known as supervised learning. At that first moment, data was entered with the aim of creating a universe of information that enables the system to establish relations between the parameters provided in an assisted way. The database is a critical factor, as well as the choice of parameters. The entire result structure is built on the premise that the data is true and reliable. Data entry was sufficient for the system to learn how to categorize and create groups. Parameters such as diameter, shape, volume and fluorescence are used for the qualification of our samples, comprised of different cell types. The machine learning process takes place based on the structure Of the data provided, and through the creation of generalization groups. In this case, data is entered into the system, causing the machine to begin to establish new connections between these.

The machine learning algorithm used in images 8A, 8B, 8B and 8D automatically infers the rules for discriminating classes, which can be applied for the complete data set. Unlike supervised approaches, unsupervised methods allow the exploitation of unknown phenotypes. The system is an essential part of the process and is used for screening images where cellular morphology is described with the help of fluorescent markers, and the use of holography. Our system learning process is based on classifications by means of defining phenotypes according to representative examples. The algorithm converts the images into a database suitable for system processing. The objects are described by the quantitative characteristics. Through the algorithm, it was possible to establish a control for the self-focus system, improving the image in terms of pixel settings. Moreover, we added the disregarding of factors such as mechanical instability, variable illumination of external sources, imperfections on the lenses and blades where the samples are deposited, which are considered pollution. We define the objects of interest to form the basis for classification. The detection of objects is based on the characteristics of these, such as the contour and properties of the region. The segmentation needs to be made before the description of the quantitative characteristics because the distinction of the components will be made through a classification algorithm. Increasing dimensionality makes the classification process exponentially more complex, since the number of features defines multidimensionality. Thus, the results depend so heavily on the proper choice of parameters. The multidimensionality of the system can be reduced by using an algorithm that automatically selects the most relevant parameters.

LED is an alternative light source in the device configuration, and was used to obtain figure 8B. The online configuration was used for this capture.

The quality of the images should be high enough and not redundant for the software system to be able to make comparisons. For this, more than one measuring parameter is used. To minimize phenotype classification errors and reduce false-negative, a total error performance metric was adopted. Regardless of the learning algorithm, an increase in the number of functionalities usually requires more training examples.

Figure 9 shows a possible type of relationship enabled by the hardware and software set. Patient A makes use of medicine X, Y, and Z. A second patient B also uses X, Y, and Z. Many times, certain effects are caused by the combined use of a group of drugs, in this case X, Y, and Z, that are not recognized precisely because there is no obvious association between them, and, of course, given the plurality of medications and combinations. By using this system, it is possible to establish links between the combined use of medicinal products and the effects resulting from them. In order to detect unexpected patterns within the sample space, i.e., anomalies, unsupervised learning is used. Generalizations are made based on detected variations. The general goal of any machine learning method is to generalize from some training examples to make accurate predictions about large sets of data samples, which have not been analyzed by the system.

**Claims**

1. Portable diagnostic test **characterized by** a system designed for capturing and analyzing images of samples of blood, fluids, and components that may exist in the human body, comprising: an artificial intelligence system; software for the conversion of image data and reconstruction of images; a database; a camera; at least one lens between the specimen and the lens of the camera; a rigid device containing at least one source of coherent light in its interior, oriented to the place where the sample is deposited.

2. Portable diagnostic test, as per claim 1, **characterized by** the fact that there is one coherent light source within it, which passes through the blood sample or other component that may be present within the human body.

3. Portable diagnostic test, as per claim 1, **characterized by** the fact that there are two coherent light sources inside the device, arranged in such a way that their beams intersect at a controlled point, which is the deposit site for the sample.

4. Portable diagnostic test, as per claim 1, **characterized by** the fact that two coherent light sources pass through the blood sample, and the beam of light coming from one of the sources is reflected at least once before reaching the sample while the other beam follows a rectilinear trajectory.

5. Portable diagnostic test, as per claim 1, **characterized by** the fact that there is, at least, one filter to decrease the power of the light present within the rigid housing.

6. Portable diagnostic test, as per claim 1, **characterized by** the fact that there is at least one removable and replaceable filter between the sample and the lens.

7. Portable diagnostic test, as per claim 1, **characterized by** the fact that a lens was inserted directly onto the blade that is laid on the sample.

8. Portable diagnostic test, as per claim 1, **characterized by** the fact that the place of insertion of the sample is a slide prepared with chemical markers.

9. Portable diagnostic test, as per claim 1, **characterized by** the fact that instead of a slide to deposit the sample, there is a blade with absorbing material, containing chemical markers.

10. Portable diagnostic test, as per claim 1, **characterized by** the fact that the application is specific for the detection of circulating tumor cells (CTCs).

11. Portable diagnostic test, as per claim 1, **characterized by** the fact that the application is specific for monitoring prostate cancer cases.

12. Portable diagnostic test, as per claim 1, **characterized by** the fact that the application is specific for qualitative urinalysis.

13. Portable diagnostic test, as per claim 1, **characterized by** the fact that there is, at least, one mirror or reflecting object between the light source and the sample.

14. Portable diagnostic test, as per claim 1, **characterized by** the fact that there is a Wi-Fi connection between the device and a tablet, smartphone or smartwatch, laptop, or computer.

15. Portable diagnostic test, as per claim 1, **characterized by** the fact that there is a Bluetooth connection between the device and a tablet, smartphone or smartwatch, notebook or computer.

16. Portable diagnostic test, as per claim 1, **characterized by** the fact that the device uses a chip inside to record and stream, or simply record, or simply stream the data.

17. Portable diagnostic test, as per claim 1, **characterized by** the fact that the blades in contact with the sample are made of polymeric material.

18. Portable diagnostic test, as per claim 1, **characterized by** the fact that blood components are tested alone.

19. Portable diagnostic test, as per claim 1, **characterized by** the fact that short or long batteries are used.

20. Portable diagnostic test, as per claim 1, **characterized by** the fact that the sample is placed under proper conditions for the simultaneous capture of data relating to fluorescence, and to holographic reconstruction, comprising: at least one light source, a lens set, at least one mirror, at least one filter, and blades - with or without chemical markers.

21. Portable diagnostic test, as per claim 1, **characterized by** the fact that it features a specific application for the detection of circulating tumor cells (CTCs).

22. Portable diagnostic test, as per claim 1, **characterized by** the fact that prepared blades replace the glass for the deposit of the sample.

23. Process to monitor the health of an individual through its continuous use, **characterized by**: sending images of blood tests to the artificial intelligence or machine learning system, via any internet-enabled device; comparing these with other images in the database; collecting information on anomalies, and similarities; sending qualitative and quantitative reports with the results.

24. Portable diagnostic test, as per claim 23, **characterized by** the fact that a database other than the history of the patient is used to track the evolution of CTCs, comprising: sending images, reconstructing images, cross referencing them with the database, and with a proper history; then sending the results to a device which may be a computer, tablet, smartphone, smartwatch, or even a device made with the specific purpose of generating alerts in case of any change.

25. Portable diagnostic test, as per claim 23, **characterized by** the fact that there is a causal correlation between the combined use of drugs, and the consequences thereof, including the predisposition to the development of diseases.

**26.** Process to analyze images, **characterized by**: placing the sample in the device described in claim 1, whereas such sample can be blood, fluid, or other component that may exist in the human body; sending the images to be processed to a remote computer or to the cloud, using the app or e-mail; processing the produced image by means of a holographic system; comparing it to other results using a remote database that may be in the cloud or on a physical server; using a machine learning system or similar to seek anomalies; then sending the results to the user.

**27.** Use of the portable diagnostic test, as per claims 1 to 22, **characterized by** the processes described in claims 23 to 26, for diagnostic analysis purposes, by means of: detecting and monitoring the evolution of Circulating Tumor Cells (CTCs); quantitative and qualitative analysis of the various blood components, including but not limited to, red and white blood cells; quantitative and qualitative analysis of body fluids and other components that may be present in the human body; examining urine for applications pertaining to the monitoring of prostate cancer.

FIGURE 1A

FIGURE 1B

FIGURE 1C

FIGURE 2

Positioning the sample on the gadget

Capturing image with light source No. 1

Submission

Transforming image into data

Reconstructing image

Capturing image with light source No. 2

Comparing it to a database

Checking similarities and anomalies

Sending it to an artificial intelligence system

Output

FIGURE 3

FIGURE 4

Did you ingest any
of these
substances?

Aspirin

Vitamin C

Dipyrone

FIGURE 5A

Pre-conditions for
running the tests

Fasting for the
last 08 hours

Fasting for the
last 06 hours

No alcohol
intake for the
last 04 hours

FIGURE 5B

Results

Lymphocytes

Basophils

Eosinophils

Red blood

cells

FIGURE 5C

History of
Diseases

Diabetes?

Yes        No

Cancer?

Yes        No

FIGURE 5D

FIGURE 6A

FIGURE 6B

FIGURE 6C

FIGURE 7A

FIGURE 7B

FIGURE 7C

FIGURE 7D

FIGURE 8

## INTERNATIONAL SEARCH REPORT

| International application No. |
| --- |
| PCT/BR2017/000124 |

| A. CLASSIFICATION OF SUBJECT MATTER |
| --- |
| IPC: G03H 1/00 (2006.01), G03H 1/04 (2006.01), G01N 15/02 (2006.01) |
| CPC:G03H 2001/005, G03H 1/0404, G03H 2001/0413, G03H 2001/0447, G03H 2001/0471, G01N 2015/0233 |
| According to International Patent Classification (IPC) or to both national classification and IPC |

| B. FIELDS SEARCHED |
| --- |
| Minimum documentation searched (classification system followed by classification symbols) |
| **CPC: G03H, G01N** |
| Documentation searched other than minimum documentation to the extent that such documents are included in the fields searched |
| **BASE DE PATENTES DO INPI-BR (SINPI)** |
| Electronic data base consulted during the international search (name of data base and, where practicable, search terms used) |
| **GOOGLE PATENTS, THOMSON REUTERS** |

| C. DOCUMENTS CONSIDERED TO BE RELEVANT | | |
| --- | --- | --- |
| Category* | Citation of document, with indication, where appropriate, of the relevant passages | Relevant to claim No. |
| X | WO 2011049965 A1 ( OZTOPRAK CHETIN [US])<br>28 April 2011 (2011-04-28)<br>Paragraphs 18, 35 - 49 and 61 - 63<br>Figures 3 and 5 | 2 – 10, 12– 17,<br>19 – 22 |
| Y | | 1, 11, 27 |
| X | WO 2013080163 A1 (CSIR [ZA])<br>06 June 2013 (2013-06-06)<br>pages 10 (lines 8 -29), 11 (lines 7 - 15),<br>15 (lines 6 - 34), 16 (lines 1 - 34) and 17 (lines 1 - 29)<br>Figures 5 and 6 | 2 – 10, 12 – 15,<br>18, 19, 21,<br>23 – 26 |
| Y | | 1, 11, 27 |
| A | CN 105974765 A (UNIV ZHEJIANG)<br>28 September 2016 (2016-09-28)<br>The whole document | |

| [X] Further documents are listed in the continuation of Box C. | [X] See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 09/01/2018 | 18/01/2018 |

| Name and mailing address of the ISA/ **BR** | Authorized officer |
| --- | --- |
| *I N P I*   Rua Sao Bento nº 1, 17º andar<br>cep: 20090-010, Centro - Rio de Janeiro/RJ<br>Facsimile No.   +55 21 3037-3663 | **Marcos Eduardo de Oliveira**<br><br>Telephone No.   +55 21 3037-3493/3742 |

Form PCT/ISA/210 (second sheet) (January 2015)

**INTERNATIONAL SEARCH REPORT**

International application No.

PCT/BR2017/000124

| C (Continuation). | DOCUMENTS CONSIDERED TO BE RELEVANT | |
|---|---|---|
| Category* | Citation of document, with indication, where appropriate, of the relevant passages | Relevant to claim No. |
| A | US 2012248292 AI ( UMV CALIFÓRNIA [US]) 04 october 2012 (201240-04) The whole document | |
| A | US 2016131882 AI (CALIFÓRNIA INST OF TECKN [US]) 12 may 2016 (2016-05-12) The whole document | |

Form PCT/ISA/210 (continuation of second sheet) (January 2015)

INTERNATIONAL SEARCH REPORT
Information on patent family members

| International application No. |
| --- |
| PCT/BR2017/000124 |

| | | | |
| --- | --- | --- | --- |
| WO 2011049965 A1 | 2011-04-28 | CA 2778284 A1 | 2011-04-28 |
| | | EP 2491366 A1 | 2012-08-29 |
| | | EP 3136079 A1 | 2017-03-01 |
| | | ES 2623375 T3 | 2017-07-11 |
| | | JP 2013508775 A | 2013-03-07 |
| | | JP 5639654 B2 | 2014-12-10 |
| | | JP 2015072481 A | 2015-04-16 |
| | | JP 5999783 B2 | 2016-09-28 |
| | | KR 20120071405 A | 2012-07-02 |
| | | US 2012218379 A1 | 2012-08-30 |
| | | US 9007433 B2 | 2015-04-14 |
| WO 2013080163 A1 | 2013-06-06 | CN 104115074 A | 2014-10-22 |
| | | CN 104115075 A | 2014-10-22 |
| | | EP 2786212 A1 | 2014-10-08 |
| | | EP 2786213 A1 | 2014-10-08 |
| | | JP 2015505983 A | 2015-02-26 |
| | | JP 2015505984 A | 2015-02-26 |
| | | MX 2014006552 A | 2014-10-06 |
| | | MX 336678 B | 2016-01-27 |
| | | MX 2014006555 A | 2014-07-22 |
| | | MX 345972 B | 2017-02-28 |
| | | US 2014327944 A1 | 2014-11-06 |
| | | US 2014365161 A1 | 2014-12-11 |
| | | WO 2013080164 A1 | 2013-06-06 |
| CN 105974765 A | 2016-09-28 | None | |
| US 2012248292 A1 | 2012-10-04 | US 8866063 B2 | 2014-10-21 |
| US 2016131882 A1 | 2016-05-12 | US 2017219999 A1 | 2017-08-03 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Quantitative Imaging with a Mobile Phone Microscope. *PLOS One,* 2014, vol. 9 (5, www.plosone.org
- **PHAM et al.** Tests in Real Time Blood Testing Using Quantitative Phase Imaging. *PLOS One,* 2013, vol. 8, www.plusone.org
- Phone Based Clinical Microscopy for Global Health Applications. *PLOS One,* 2009, vol. 4 (7